# EUROPEAN PATENT SPECIFICATION

(11) **EP 3 784 182 B1**
(45) Date of publication and mention of the grant of the patent: **18.12.2024**
(21) Application number: 19721463.8
(22) Date of filing: 15.04.2019
(51) Int. Cl.: A61F 13/05

(54) **DRESSING PROVIDING APERTURES WITH MULTIPLE ORIFICE SIZES FOR NEGATIVE-PRESSURE THERAPY**
VERBAND MIT ÖFFNUNGEN MIT MEHREREN ÖFFNUNGSGRÖSSEN FÜR DIE UNTERDRUCKTHERAPIE
PANSEMENT FOURNISSANT DES OUVERTURES AVEC DE MULTIPLES TAILLES D'ORIFICE POUR UNE THÉRAPIE PAR PRESSION NÉGATIVE

(30) Priority: 23.04.2018 US 201815960310
(43) Date of publication of application: 03.03.2021
(73) Proprietor: Solventum Intellectual Properties Company, Maplewood, MN 55144 (US)
(72) Inventor: LOCKE, Christopher, Brian, San Antonio TX 8265-9508 (US); ROBINSON, Timothy, Mark, San Antonio TX 78265-9508 (US); ALLEN, Diwi, L., San Antonio TX 78265-9508 (US); KHARKAR, Prathamesh, Madhav, San Antonio TX 78265-9508 (US); SCHMIDT, Marisa, San Antonio TX 78265-9508 (US); RICE, Justin, San Antonio TX 78265-9508 (US); INGRAM, Shannon, C., San Antonio TX 78265-9508 (US); SMITH, Kenneth, R., San Antonio TX 78265-9508 (US); PRATT, Benjamin, Andrew, San Antonio TX 78265-9508 (US); CARROLL, Christopher, Allen, San Antonio TX 78265-9508 (US)
(74) Representative: Simmons & Simmons
(86) International application number: PCT/US2019/027463
(87) International publication number: WO 2019/209562

(56) References cited:
- EP-A1- 2 098 257
- EP-B1- 2 098 257
- WO-A1-2013/129343
- DE-A1- 102006 017 194
- DE-A1- 102006 017 194
- GB-A- 2 377 939
- US-A1- 2017 189 237

## Description

### TECHNICAL FIELD

The invention set forth in the appended claims relates generally to tissue treatment systems and more particularly, but without limitation, to apparatus for treating a tissue site.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

WO2013/129343A1, DE102006017194A1, US2017/189237A1, EP2098257A1, and GB2377939A each discuss wound dressings.

### BRIEF SUMMARY

There is provided an apparatus for treating a tissue site which comprises a contact layer formed from a compressible material. The contact layer comprises a first surface, a second surface, and a plurality of apertures extending at least partially through the contact layer. At least a portion of the apertures include a first plurality of orifices in the first surface having a diameter in a first diameter range and at least a portion of the apertures include a second plurality of orifices in the second surface having a diameter in a second diameter range. The contact layer also comprises a cover configured to form a sealed space including the contact layer and the tissue site. The first diameter range is from about 2 mm to about 6 mm. The second diameter range is from about 8 mm to about 15 mm. In use, either the first surface or the second surface of the contact layer contacts the tissue site. Optional features are set out in the dependent claims.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Embodiments described with reference to Figures 1 to 5 fall within the scope of the appended claims. The remaining figures relate to embodiments which are useful for understanding the claimed subject matter but are not within the scope of the claimed subject matter.
Figure 1 is a schematic diagram of an example embodiment of a therapy system that can provide negative-pressure therapy in accordance with this specification;
Figure 2 is a simplified cutaway view of an example embodiment of a contact layer that may be associated with the therapy system of Figure 1;
Figure 3 is a simplified cutaway view of another example embodiment of a contact layer that may be associated with the therapy system of Figure 1;
Figure 4 is a simplified cutaway view of another example embodiment of a contact layer that may be associated with the therapy system of Figure 1;
Figure 5 is a simplified cutaway view of another example embodiment of a contact layer that may be associated with the therapy system of Figure 1;
Figure 6 is a simplified cutaway view of another example embodiment of a contact layer that may be associated with the therapy system of Figure 1;
Figure 7 is another simplified cutaway view of the example embodiment of the contact layer of Figure 6;
Figure 8 is a simplified perspective view of an example embodiment of a contact layer that may be associated with the therapy system of Figure 1;
Figure 9 is another simplified perspective view of the example embodiment of the contact layer of Figure 8;
Figure 10 is another simplified perspective view of the example embodiment of the contact layer of Figure 8;
Figure 11 is a simplified planar view of another example embodiment of a contact layer that may be associated with the therapy system of Figure 1;
Figure 12 is a simplified perspective view of the embodiment of Figure 11;
Figure 13 is a simplified planar view of another example embodiment of a contact layer that may be associated with the therapy system of Figure 1;
Figure 14 is a simplified perspective view of the embodiment of Figure 13;
Figure 15 is a simplified planar view of another example embodiment of a contact layer that may be associated with the therapy system of Figure 1;
Figure 16 is a simplified perspective view of the embodiment of Figure 15;
Figure 17 is a simplified planar view of another example embodiment of a contact layer that may be associated with the therapy system of Figure 1;
Figure 18 is a simplified perspective view of the embodiment of Figure 17;
Figure 19 is a simplified planar view of another example embodiment of a contact layer that may be associated with the therapy system of Figure 1;
Figure 20 is a simplified perspective view of the embodiment of Figure 19;
Figure 21 is a simplified planar view of another example embodiment of a contact layer that may be associated with the therapy system of Figure 1;
Figure 22 is a simplified perspective view of the embodiment of Figure 21;
Figure 23 is a simplified planar view of another example embodiment of a contact layer that may be associated with the therapy system of Figure 1;
Figure 24 is a simplified perspective view of the embodiment of Figure 23;
Figure 25 is a simplified perspective view of a kit including a contact layer; and
Figure 26 is another simplified perspective view of a kit including a contact layer.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

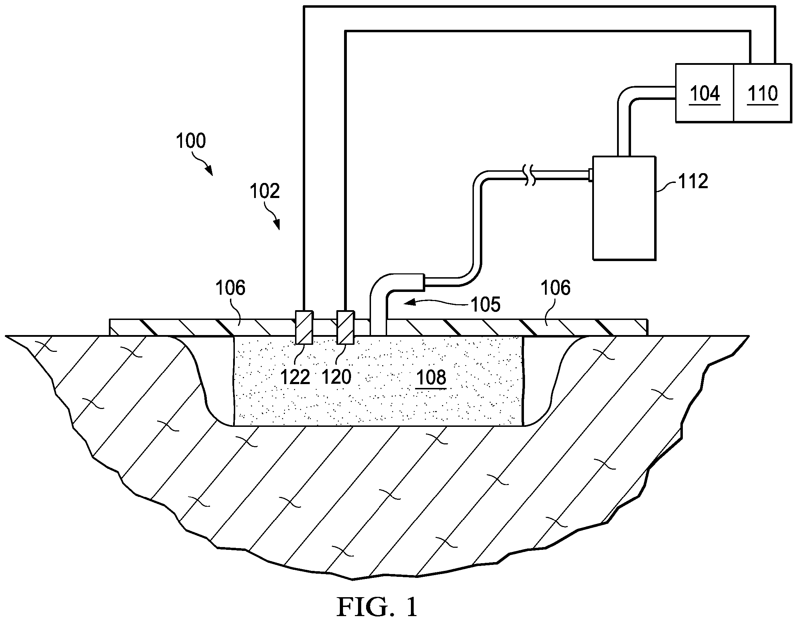

Figure 1 is a simplified schematic diagram of an example embodiment of a therapy system 100 that can provide negative-pressure therapy in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a contact layer 108. A regulator or a controller, such as a controller 110, may also be coupled to the negative-pressure source 104.

In some embodiments, a dressing interface 105 may facilitate coupling the negative-pressure source 104 to the dressing 102. For example, such a dressing interface may be the SENSAT.R.A.C.^{™} Dressing available from Acelity L.P. of San Antonio, Texas. The therapy system 100 may optionally include a fluid container, such as a container 112, coupled to the dressing 102 and to the negative-pressure source 104.

Additionally, the therapy system 100 may include sensors to measure operating parameters and provide feedback signals to the controller 110 indicative of the operating parameters. As illustrated in Figure 1, for example, the therapy system 100 may include a pressure sensor 120, an electric sensor 122, or both, coupled to the controller 110. The pressure sensor 120 may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the negative-pressure source 104 may be directly coupled to the controller 110, and may be indirectly coupled to the dressing 102 through the container 112.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of a fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110 and other components into a therapy unit. A negative-pressure supply may also have one or more supply ports configured to facilitate coupling and de-coupling the negative-pressure supply to one or more distribution components.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the contact layer 108, for example. The controller 110 is also preferably configured to receive one or more input signals, such as a feedback signal, and programmed to modify one or more operating parameters based on the input signals.

Sensors, such as the pressure sensor 120 or the electric sensor 122, are generally known in the art as any apparatus operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the negative-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 300 g/m² per twenty-four hours in some embodiments. In some example embodiments, the cover 106 may be a polymer drape, such as a polyurethane film, that is permeable to water vapor but impermeable to liquid. Such drapes typically have a thickness in the range of 25-50 microns. For permeable materials, the permeability generally may be low enough that a desired negative pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

### Contact Layer

The contact layer 108 can be generally adapted to contact a tissue site. The contact layer 108 may be partially or fully in contact with the tissue site. If the tissue site is a wound, for example, the contact layer 108 may partially or completely fill the wound, or may be placed over the wound. The contact layer 108 may take many forms, and may have many sizes, shapes, or thicknesses depending on a variety of factors, such as the type of treatment being implemented or the nature and size of a tissue site. For example, the size and shape of the contact layer 108 may be adapted to the contours of deep and irregular shaped tissue sites. Moreover, any or all of the surfaces of the contact layer 108 may have projections or an uneven, course, or jagged profile that can induce strains and stresses on a tissue site, which can promote granulation at the tissue site.

In some embodiments, the contact layer 108 may comprise or consist of two substantially planar surfaces and a depth or thickness orthogonal to the planar surfaces. The contact layer 108 comprises a first surface and a second surface. The first surface and/or second surface may have a surface area from about 1 cm² to about 400 cm², or from about 2 cm² to about 200 cm², or from about 4 cm² to about 100 cm².

In some embodiments, the contact layer 108 may comprise or consist of a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site. In some illustrative embodiments, the pathways of a manifold may be interconnected to improve distribution or collection of fluids across a tissue site.

In some embodiments, the contact layer 108 may be formed from a suitably compressible material. For example, in some illustrative embodiments, the contact layer 108 may comprise or consist of a porous foam material having interconnected cells or pores. For example, the contact layer 108 may comprise or consist of cellular foam such as open-cell foam. The open-cell foam may be reticulated foam. Liquids, gels, and other foams may also include or be cured to include fluid pathways. In some embodiments, the contact layer 108 may comprise projections that form interconnected fluid pathways. For example, the contact layer 108 may be molded to provide surface projections that define interconnected fluid pathways. In some embodiments, the foam may have an average pore size that varies according to needs of a prescribed therapy. For example, in some embodiments, the contact layer 108 may be foam having pore sizes in a range of 400-600 microns. In some embodiments, the contact layer 108 may have a tensile strength that also varies according to needs of a prescribed therapy.

In some embodiments, the foam material of the contact layer 108 may be characterized with respect to density. For example, in some embodiments, the contact layer 108 may be characterized as a relatively dense material. In various embodiments, the contact layer 108 may have a density of about 24 kg/m³ to about 125 kg/m³ or about 24 kg/m³ to about 72 kg/m³.

In some embodiments, the contact layer 108 may comprise a single foam layer. In some embodiments, the contact layer 108 may comprise two or more layers that have been joined together to form the contact layer 108. The two or more sublayers may be joined together by flame lamination or by a reactive adhesive, for example, a heat reactive adhesive system such as a hot melt adhesive or a chemically reactive adhesive system such as isocyanate adhesives, epoxy adhesives, silane adhesives, or combinations thereof.

In some embodiments, the contact layer 108 may be hydrophobic. In some an embodiments, the hydrophobic characteristics may prevent the foam from directly absorbing fluid, such as wound exudate, but may allow fluid to pass through a fluid pathway. For example, in some embodiments, the foam may be polyurethane foam, a silicone foam, a polyether block amide foam, such as PEBAX^{®}, an acrylic foam, a polyvinyl chloride (PVC) foam, a polyolefin foam, a polyester foam, a polyamide foam, a thermoplastic elastomer (TPE) foam such as a thermoplastic vulcanizate (TPV) foam, or another crosslinking elastomeric foam such as foams formed from styrene-butadiene rubber (SBR) and ethylene propylene diene monomer (EPDM) rubber. For example, the contact layer 108 may comprise hydrophobic, open-cell foam. In one non-limiting example, the contact layer 108 may comprise a reticulated polyurethane foam such as the foam employed in the V.A.C.^{®} GRANUFOAM^{™} Dressing or the foam employed in the V.A.C. VERAFLO^{™} Dressing, both available from Acelity L.P., Inc. of San Antonio, Texas.

In other embodiments, the contact layer 108 may be hydrophilic. In some embodiments, the hydrophillic characteristics may be effective to wick fluid while also continuing to distribute negative pressure to the tissue site. In some embodiments, the wicking properties of the contact layer 108 may draw fluid away from the tissue site by capillary flow or other wicking mechanisms. An example of a hydrophilic foam may include a polyvinyl alcohol or polyether, open-cell foam. Other foams that may exhibit hydrophilic characteristics include hydrophobic foams that have been treated or coated to provide hydrophilicity. For example, the contact layer 108 may be a treated open-cell polyurethane foam. In one non-limiting example, the contact layer 108 may comprise a polyvinyl alcohol, open-cell foam such as the foam employed in the V.A.C. WHITEFOAM^{™} Dressing available from Acelity L.P., Inc. of San Antonio, Texas.

In some embodiments, the contact layer 108 may further promote granulation at a tissue site when pressure within a sealed therapeutic environment is reduced. For example, any or all of the surfaces of the contact layer 108 may have an uneven, coarse, or jagged profile that can induce microstrains and stresses at a tissue site if negative pressure is applied through contact layer 108.

The contact layer 108 comprises a plurality of apertures extending at least partially through the thickness of the contact layer 108. The contact layer 108 is configurable such that at least a portion of the apertures includes a first plurality of orifices having a diameter in a first diameter range, and such that at least a portion of the apertures includes a second plurality of orifices having a diameter in a second diameter range. The term "aperture" in this context broadly refers to a void space extending some depth into or through a contact layer. The term "orifice" in this context more narrowly refers to an opening to an aperture in a plane in which an aperture intersects either the first surface or the second surface of the contact layer 108.

The first diameter range is about 2 mm to about 6 mm. The second diameter range is from about 8 mm to about 15 mm. Suitable sizes for the first orifice and the second orifice may be determined based upon the particular needs of a prescribed therapy.

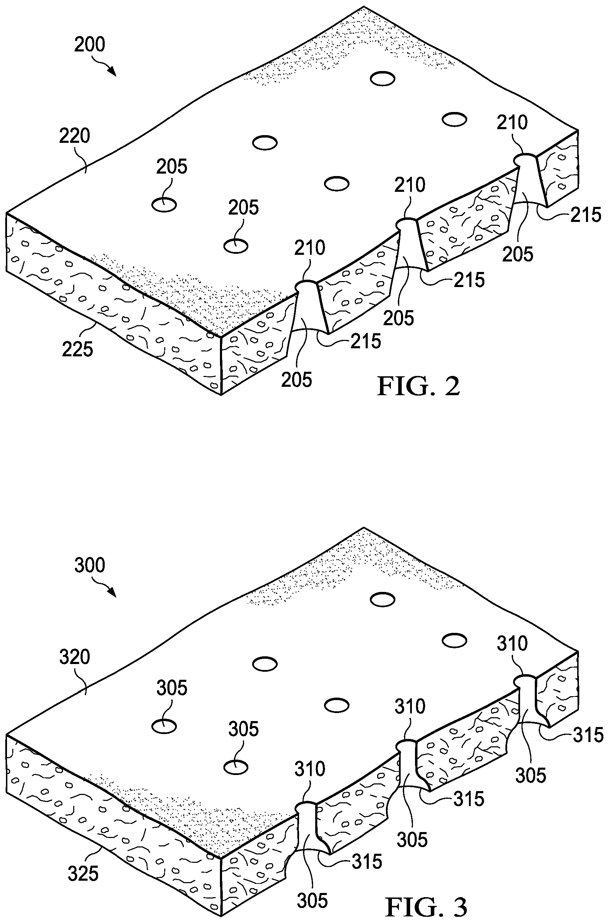

In some embodiments, a contact layer may comprise a plurality of apertures extending through the contact layer, each having an orifice within the first diameter range and an orifice within the second diameter range. For example, Figure 2 is a simplified cutaway view of an example embodiment of a contact layer 200 including a plurality of apertures 205 extending through the contact layer 200. The apertures 205 may each have a first orifice 210 having a diameter in the first diameter range and a second orifice 215 having a diameter in the second diameter range. The apertures 205 may have a suitable transitional shape between the first orifice 210 and the second orifice 215. For example, in the embodiment Figure 2, the apertures 205 have a variable diameter, and may define a conical frustum void-space. In some embodiments, the first orifices 210 may be disposed on a first surface 220 of the contact layer 200 and the second orifices 215 may be disposed on a second surface 225 of the contact layer 200.

Figure 3 is a simplified cutaway view of another example embodiment of a contact layer 300 including a plurality of apertures 305 extending through the contact layer 300 and having a first orifice 310 having a diameter in the first diameter range and a second orifice 315 having a diameter in the second diameter range. In the embodiment of Figure 3, the aperture 305 may define at least a portion of a hyperboloidic void-space, for example a "bottle-shaped" void-space. The first orifices 310 may be disposed on a first surface 320 of the contact layer 300 and the second orifices 315 may be disposed on a second surface 325 of the contact layer 300.

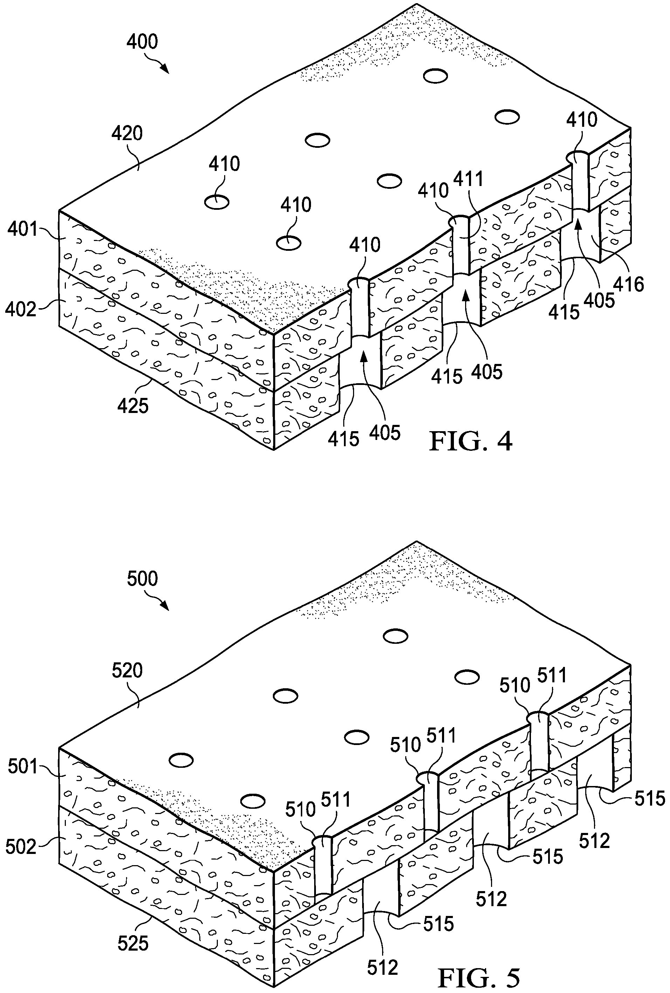

Figure 4 is a simplified cutaway view of another example embodiment of a contact layer 400 including a plurality of apertures 405 extending through the contact layer 400 and having a first orifice 410 having a diameter in the first diameter range and a second orifice 415 having a diameter in the second diameter range. The contact layer 400 may comprise a first sublayer 401 joined to a second sublayer 402. In the embodiment Figure 4, the apertures 405 include a first portion 411 extending through the first sublayer 401 and a second portion 416 extending through the second sublayer 402. As shown in the example of Figure 4, the first portion 411 may be narrower than the second portion 416. The first orifices 410 may be disposed on a first surface 420 of the contact layer 400 and the second orifices 415 may be disposed on a second surface 425 of the contact layer 400.

A contact layer comprises a plurality of apertures extending only partially through the contact layer. A first portion of the apertures may extend from a first surface and have orifices within the first diameter range. A second portion of the apertures may extend from a second surface and have orifices within the second diameter range. Figure 5 is a simplified cutaway view of another example embodiment of a contact layer 500 comprising a first sublayer 501 joined to a second sublayer 502. A first plurality of apertures 511 having orifices within the first diameter range may extend through the first sublayer 501. A second plurality of apertures 512 having orifices within the second diameter range may extend through the second sublayer 502. The first plurality of apertures 511 may be offset with respect to the second plurality of apertures 512, for example, such that the first plurality of apertures 511 and the second plurality of apertures 512 each extend only partially through the contact layer 500. The first orifices 510 may be disposed on a first surface 520 of the contact layer 500 and the second orifices 515 may be disposed on a second surface 525 of the contact layer 500.

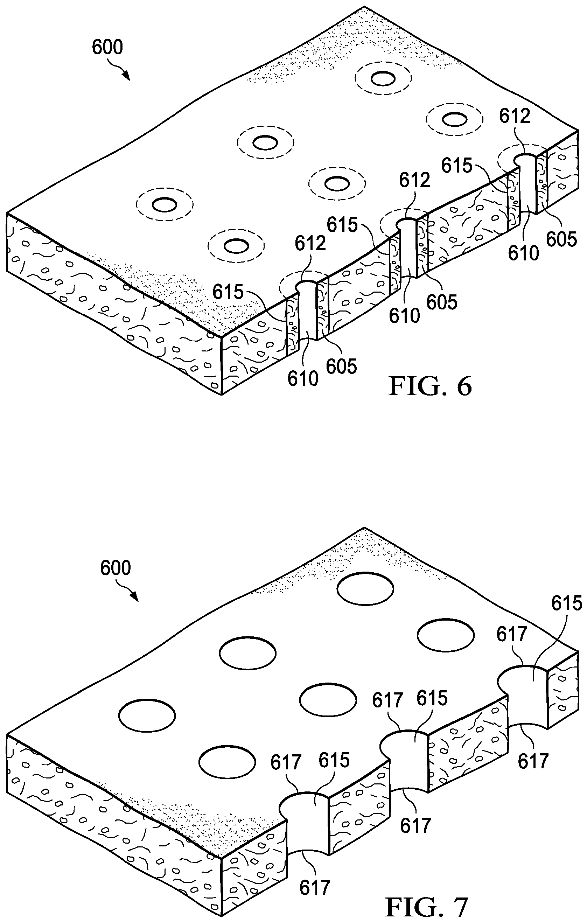

In some embodiments, a contact layer may comprise a plurality of removable portions. The plurality of removable portions may have apertures including orifices in a first diameter range. The removable portions may be removable from the contact layer to form apertures including orifices in a second diameter range. Figure 6 is a simplified cutaway view of another example embodiment of a contact layer 600. The contact layer 600 may comprise a plurality of removable portions 605. Each of the removable portions may include a first aperture 610 having first orifices 612 in the first diameter range. Each of the removable portions 605 may be fitted within a second aperture 615 of the contact layer 600. In some embodiments, the removable portions 605 may comprise a material separate from the contact layer 600 that is inserted within the second apertures 615 and held in place, for example, by friction. In some, other embodiments, the removable portions 605 may be formed from the material that also forms the contact layer 600. For example, in such embodiments, the removable portions 605 may be formed by cutting or perforating the contact layer 600 and leaving the removable portions 605 in place.

Figure 7 is another simplified cutaway view of the embodiment of the contact layer 600 of Figure 6, illustrating the removable portions 605 removed. In some embodiments, each of the removable portions 605 may be removable to yield the second apertures 615 having second orifices 617 in the second diameter range. In some embodiments, the first apertures 610, the second apertures 615, or both may have a substantially constant diameter. For example, each of the first apertures 610 may include two first orifices 612 in the first diameter range and each of the second apertures 615 may include two second orifices 617 in the second diameter range.

In some, other embodiments, the first apertures 610, the second apertures 615, or both may have a diameter that varies over the thickness of the contact layer 600. For example, each of the first apertures 610 may include two orifices having different diameters, each of the second apertures 615 may include two orifices having different diameters. For example, the first apertures 610, the second apertures 615, or both may define at least a portion of a conical void-space, a hyperboloidic void-space, or the like. In such embodiments, the contact layer may be configurable to provide orifices having diameters within a first diameter range, orifices having diameters within a second diameter range, orifices having diameters within a third diameter range, and orifices having diameters within a fourth diameter range.

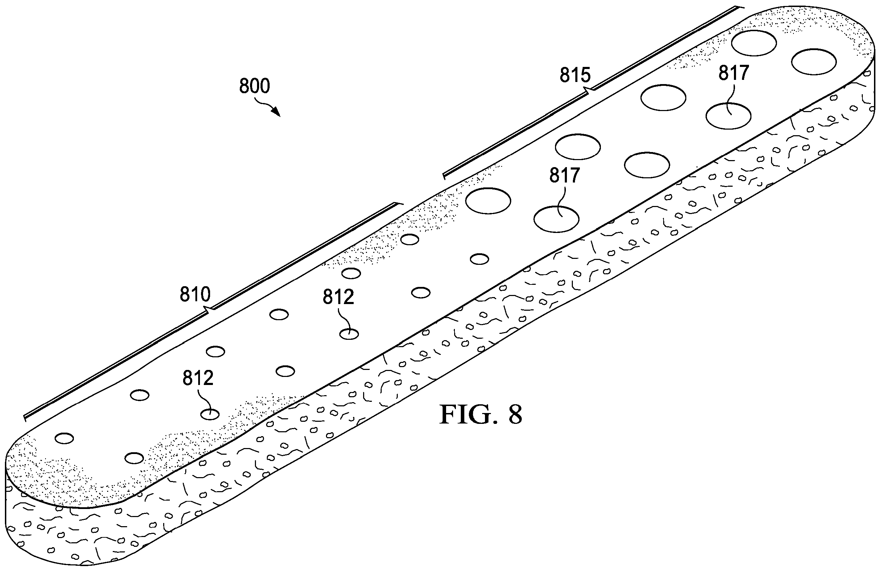

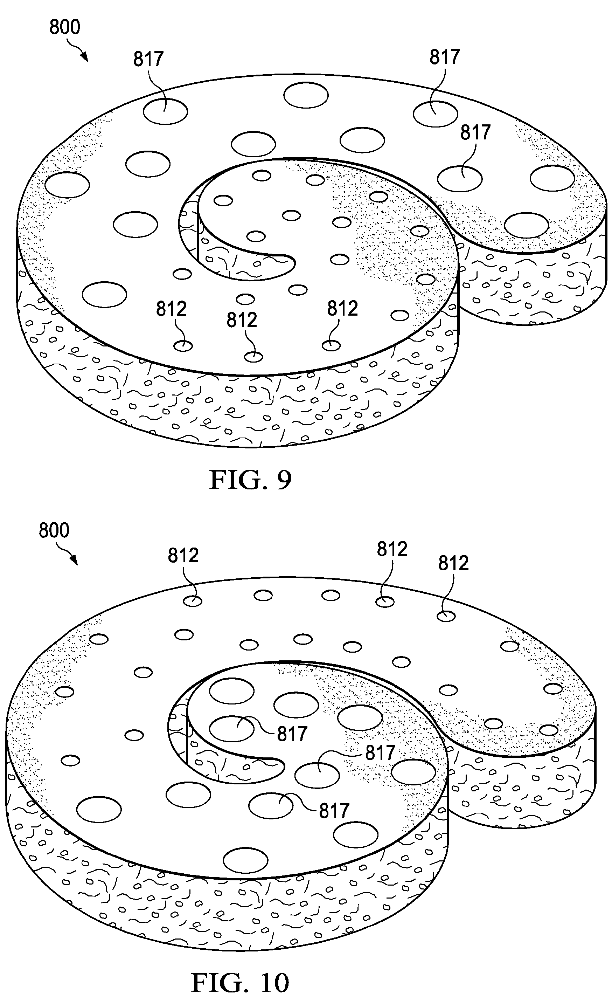

In some embodiments, a contact layer may comprise a first plurality of apertures including orifices having diameters within a first diameter range and a second plurality of apertures including orifices having diameters within a second diameter range. Figure 8 is a simplified perspective view of another example embodiment of a contact layer 800. The contact layer 800 may include a first plurality of apertures 810 and a second plurality of apertures 815. The first plurality of apertures 810 and the second plurality of apertures 815 may extend partially or entirely through the contact layer 800. Each of the first plurality of apertures 810 may include an orifice 812 having a diameter within a first diameter range and each of the second plurality of apertures 815 may include an orifice 817 having a diameter within a second diameter range. In some embodiments, the first plurality of apertures 810 may be grouped together and the second plurality of apertures 815 may also be grouped together. For example, the first plurality of apertures 810 may be generally disposed in a first portion of the contact layer 800 and the second plurality of apertures 815 may be generally disposed in a second portion of the contact layer 800. The contact layer 800 may be configurable in either a first configuration or a second configuration, for example, such that either the orifices 812 having a diameter within a first diameter range or the orifices 817 having a diameter within a second diameter range are disposed centrally with respect to the contact layer. For example, Figure 9 illustrates the contact layer 800 of Figure 8 in a first conformation in which the contact layer 800 is coiled such that the orifices 812 having a diameter within a first diameter range are disposed centrally after the contact layer 800 has been formed into a spiral. In some embodiments, the contact layer may be provided in such a spiral conformation. Also for example, Figure 10 illustrates the contact layer 800 of Figure 8 in a second conformation in which the contact layer 800 is coiled such that the orifices 817 having a diameter within a second diameter range are disposed centrally after the contact layer 800 has been formed into a spiral.

In various embodiments, the first surface and/or the second surface of the contact layer 108 may have any suitable shape, examples of which include but are not limited to, triangles, squares, rectangles, ellipses, circles, ovals, and various polygons having four, five, six, seven, eight, or more sides. The shape and area of the first surface and the second surface may be customized to the location and type of tissue site onto which the contact layer 108 is to be applied. In some embodiments, the contact layer 108 may have a thickness from about 10 mm to about 500 mm, for example, from about 10 mm to about 100, or from about 100 mm to about 200 mm, or from about 200 mm to about 300 mm, or from about 300 mm to about 400 mm, or from about 400 mm to about 500 mm.

In some embodiments, the contact layer 108 may be configurable into a one of multiple, potential sizes or shapes, as desired. For example, in some embodiments, the contact layer 108 may comprise one or more separation-lines, such as perforations, slits, splits, indentions, or the like. For example, the separation-lines may enable the contact layer 108 to be conformed to a tissue site having a particular size or shape by a user without the use of additional tools. For example, the separation-lines may enable to user to divide the contact layer 108 into various portions. In various embodiments, the separation-lines may be disposed within the contact layer 108 in any suitable pattern or combination of patterns such that, when separated along the separation-lines, one or more of the resultant portions of the contact layer 108 have a desired size and/or shape. The perforations may allow a contact layer 108 to be customized to one of multiple sizes or shapes. The contact layer 108 may offer multiple combinations of lines along which the contact layer 108 can be separated, for example, such that multiples potential size and shape combinations are possible.

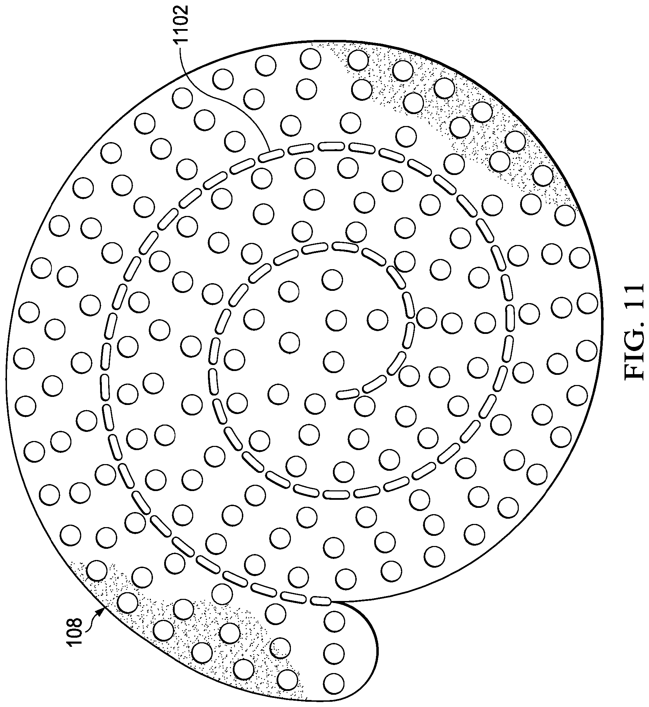

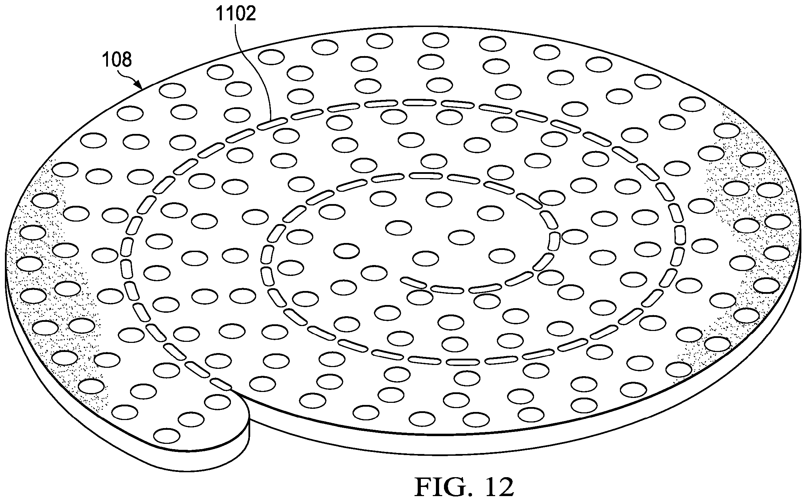

For example, in the embodiment of Figure 11 the contact layer 108 is spirally-shaped and includes perforations 1102 disposed within the contact layer 108 in a spiral. Figure 12 illustrates a perspective view of the contact layer 108 of Figure 11. The contact layer 108 may be separated along some portion of the perforations 1102 to yield a spiral having a desired size.

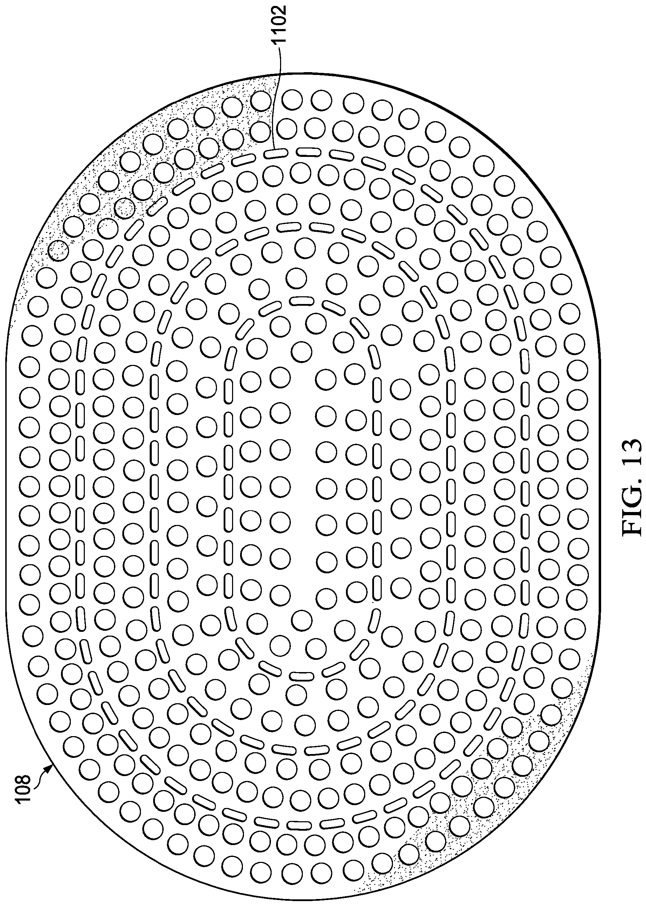

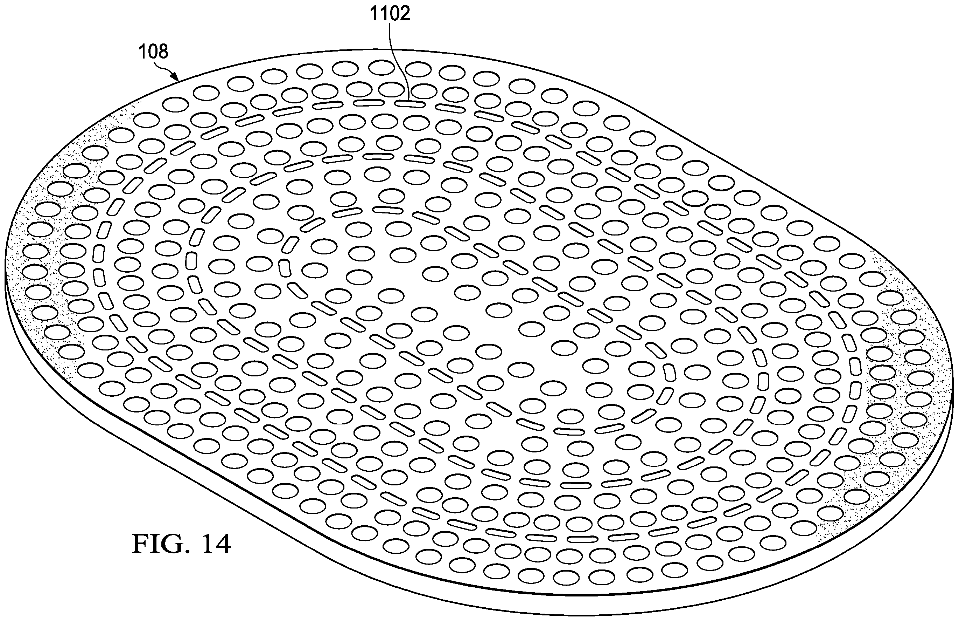

In the embodiment of Figure 13 the contact layer 108 is elliptically-shaped and includes perforations 1102 disposed within the contact layer 108 in a plurality of concentric ellipses. Figure 14 illustrates a perspective view of the contact layer 108 of Figure 13. The contact layer 108 may be separated along the perforations 1102 of one of the ellipses to yield an ellipse of a desired size.

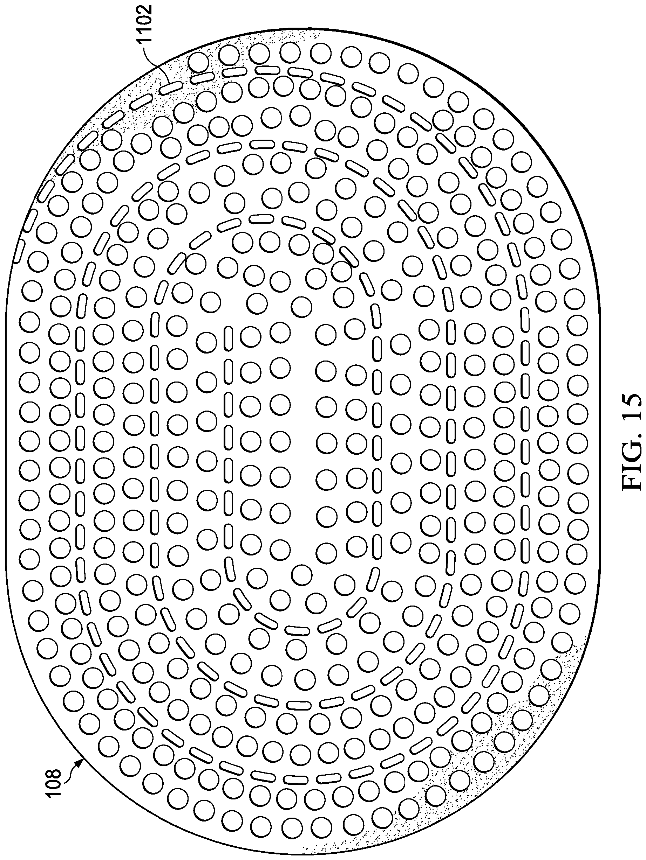

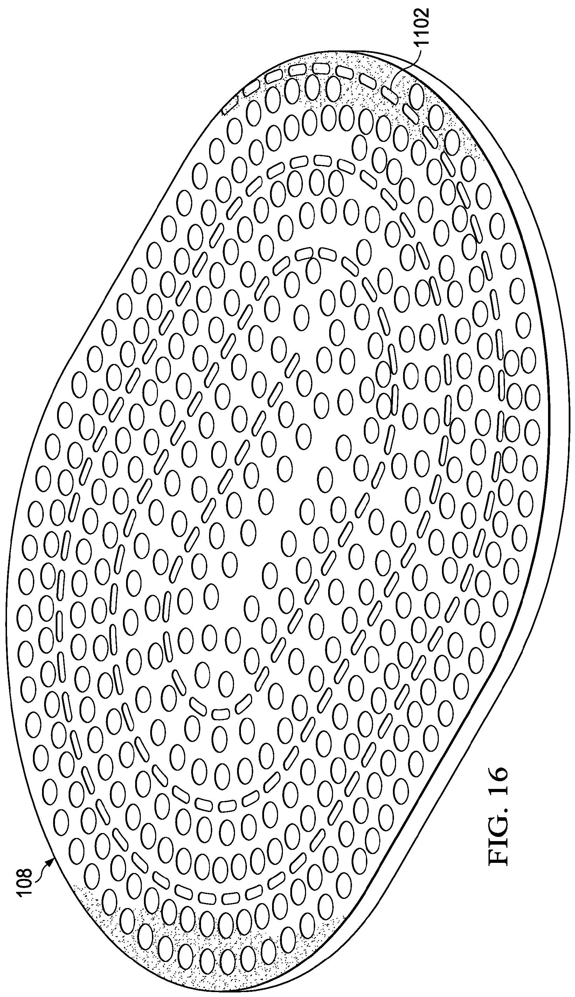

In the embodiment of Figure 15 the contact layer 108 is elliptically-shaped and includes perforations 1102 disposed within the contact layer 108 in a spiral. Figure 16 illustrates a perspective view of the contact layer 108 of Figure 15. The contact layer 108 may be separated along some portion of the perforations 1102 to yield a spiral having a desired size.

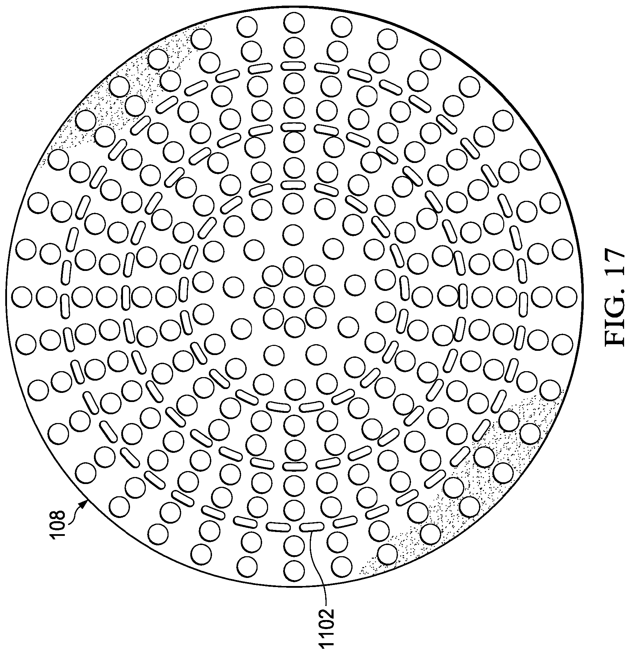

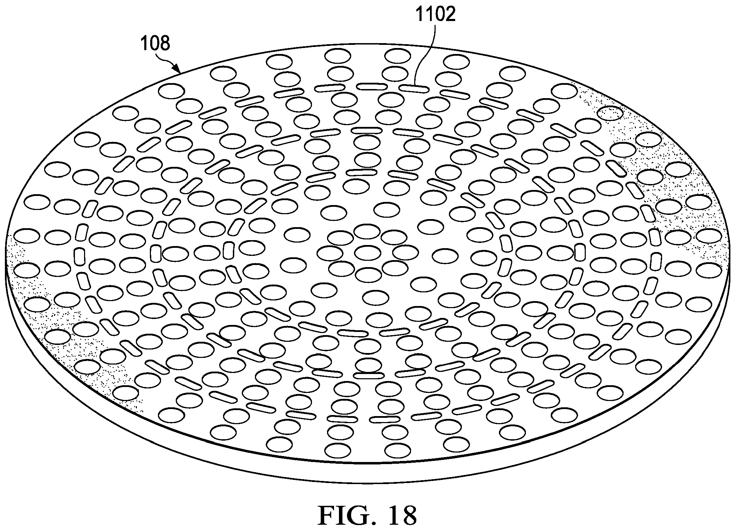

In the embodiment of Figure 17 the contact layer 108 is round and includes perforations 1102 disposed within the contact layer 108 in a plurality of concentric circles. Figure 18 illustrates a perspective view of the contact layer 108 of Figure 17. The contact layer 108 may be separated along the perforations 1102 of one of the circles to yield a circle of a desired size.

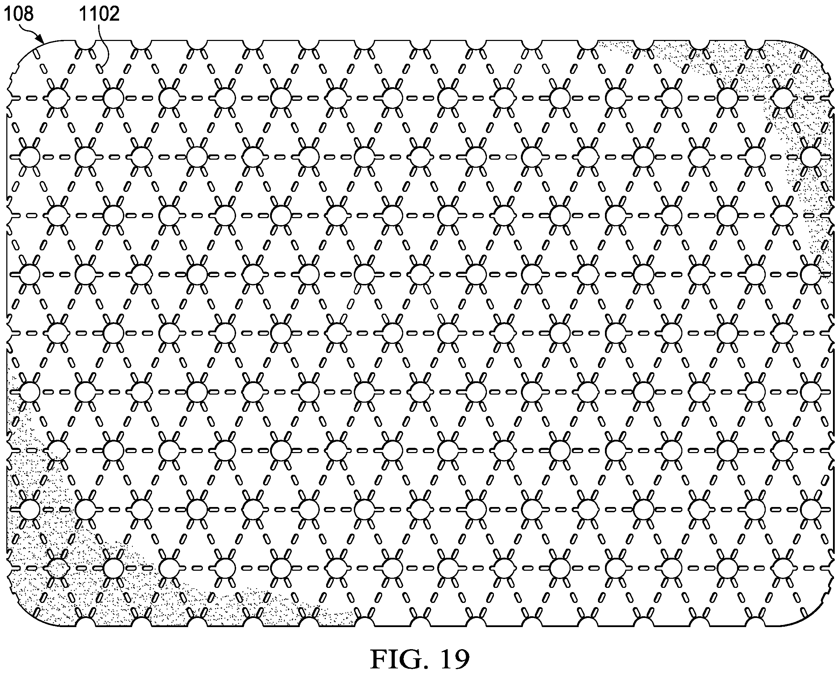

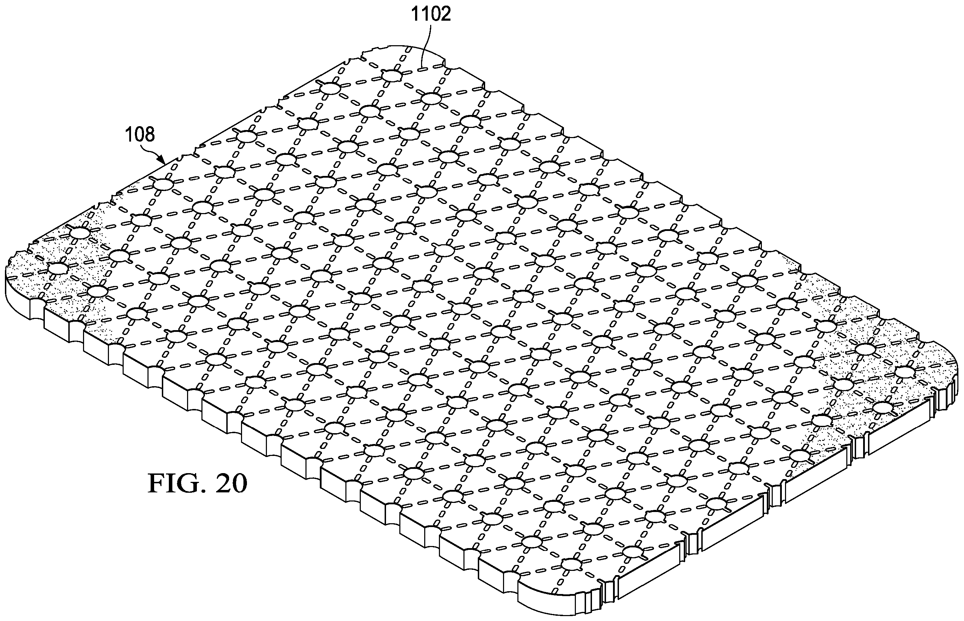

In the embodiment of Figure 19 the contact layer 108 is rectangular and includes perforations 1102 disposed within the contact layer 108 in a plurality of lines extending in multiple directions. Figure 20 illustrates a perspective view of the contact layer 108 of Figure 19. The contact layer 108 may be separated along the perforations 1102 of one or more of the lines to form a desired size and shape.

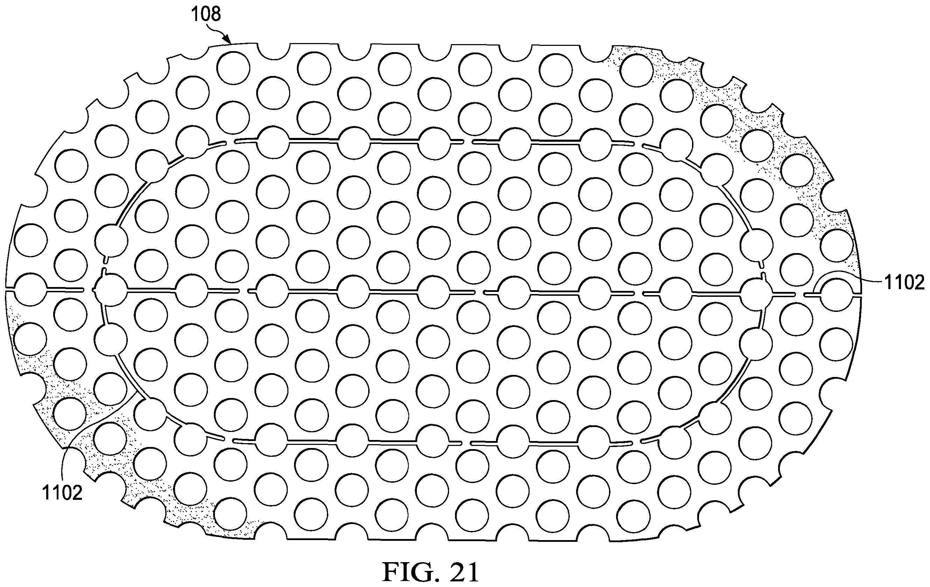

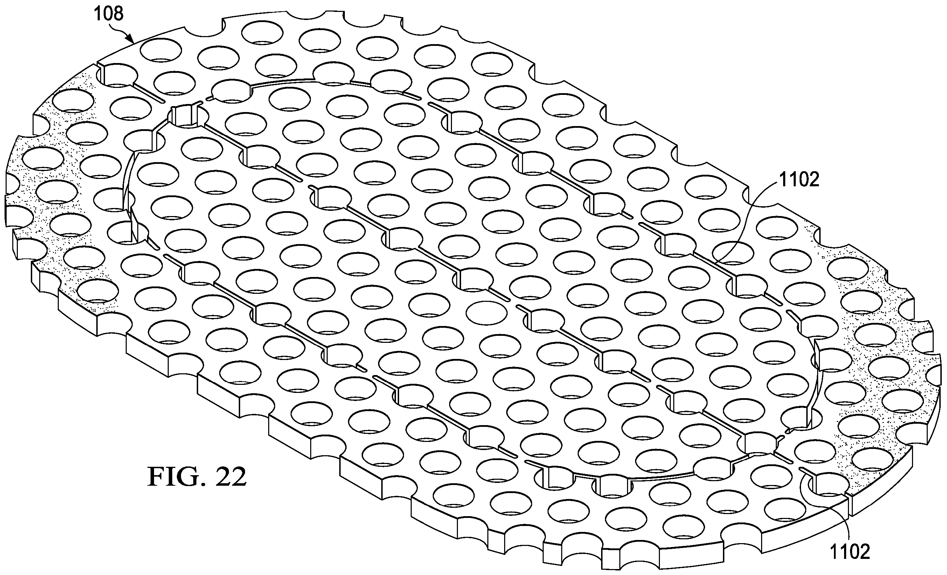

In the embodiment of Figure 21 the contact layer is an oval and includes perforations 1102 disposed within the contact layer 108 in an oval and in a line. Figure 22 illustrates a perspective view of the contact layer 108 of Figure 21. The contact layer 108 may be separated along the perforations 1102 of one or more of the lines to form a desired size and shape.

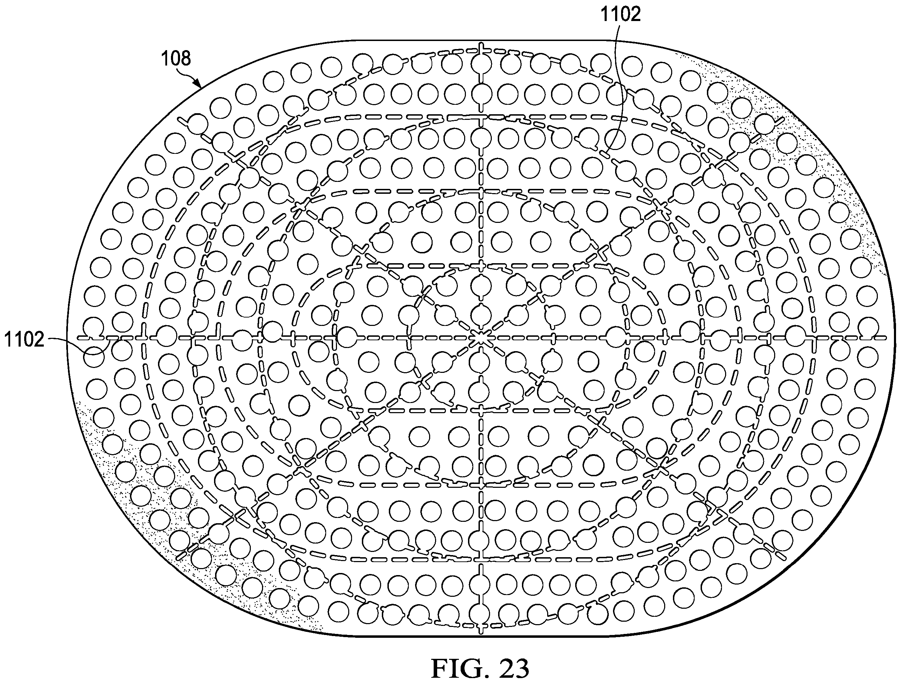

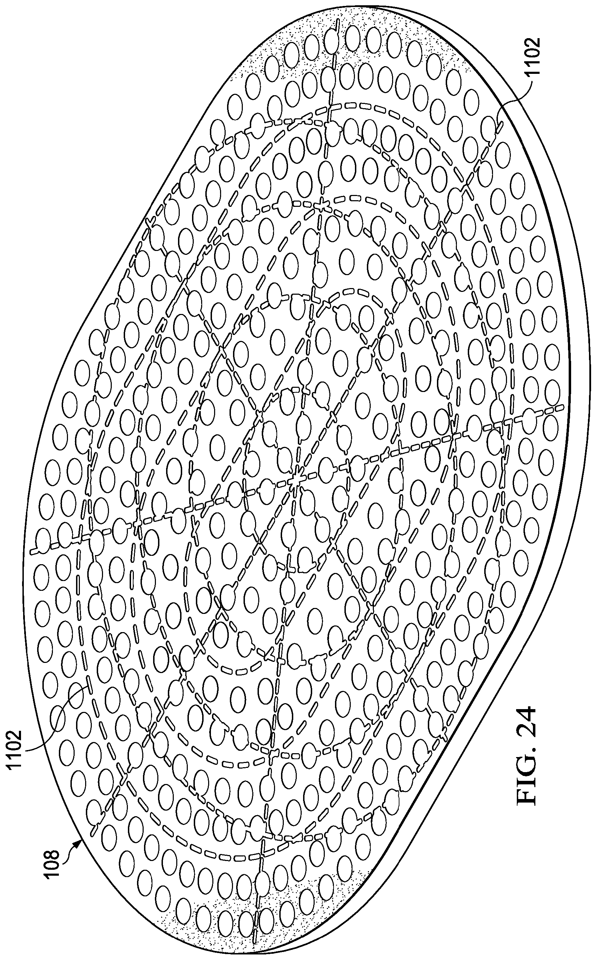

In the embodiment of Figure 23 the contact layer 108 is an oval and includes perforations 1102 disposed within the contact layer 108 in concentric circles and oval and in lines radiating from the center. Figure 24 illustrates a perspective view of the contact layer 108 of Figure 23. The contact layer 108 may be separated along the perforations 1102 of one or more of the lines to form a desired size and shape.

In some embodiments, the contact layer 108 may be provided as a part of a kit. In various embodiments, for example, the kit may include a contact layer 108, a secondary layer, a cover, or combinations thereof. Generally, a secondary layer may comprise fluid pathways interconnected so as to improve distribution or collection of fluids. For example, in some embodiments, a secondary layer may comprise or consist essentially of a porous material. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. In some examples, a secondary layer may comprise or consist essentially of reticulated polyurethane foam.

In some embodiments, the kit may include two or more contact layers 108, two or more secondary layers, or combinations thereof. The two or more contact layer 108 may vary with respect to various parameters such as thickness; density; presence, size, number, and/or distribution of apertures; or the like. For example, the kit may include a contact layer 108 having a particular property such as thickness, density, or apertures and another contact layer 108 having another property. Similarly, the two or more secondary layers may vary with respect to various properties, such as thickness, density, or porosity.

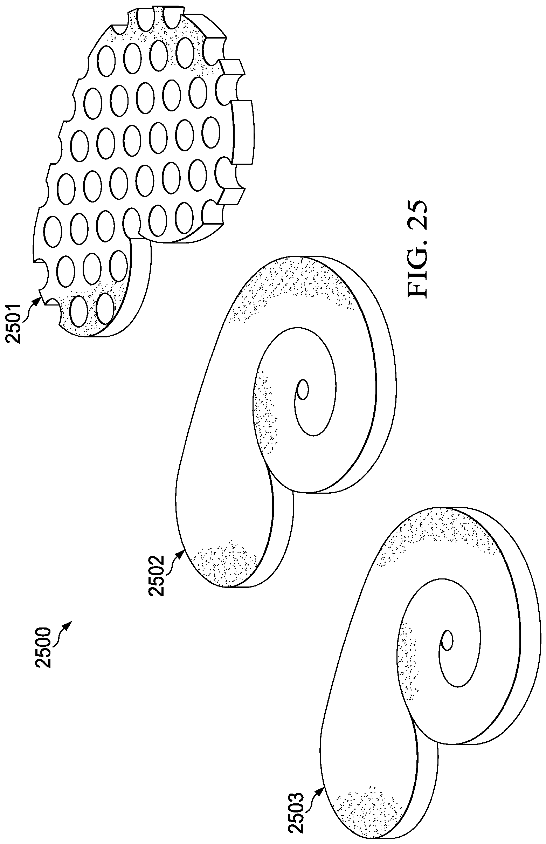

For example, Figure 25 illustrates an embodiment of a kit 2500 including a first contact layer 2501, a second contact layer 2502, and a secondary layer 2503. The first contact layer 2501 may include apertures while apertures may be absent from the second contact layer 2502. The first contact layer 2501 and the second contact layer 2502 may each include perforations, enabling the first contact layer 2501 and the second contact layer 2502 to be conformed to a portion of a tissue site. For example, in some embodiments the first contact layer 2501 may be conformed to a first portion of a tissue site and the second contact layer 2502 may be conformed to a second portion of a tissue site, depending upon the desired therapy of the tissue site.

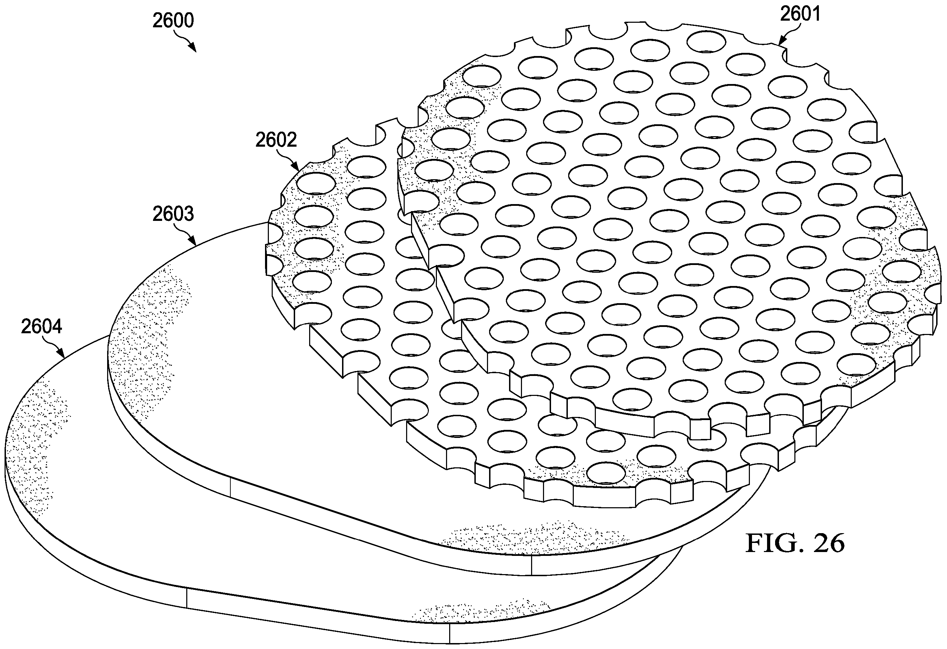

Figure 26 illustrates an embodiment of a kit 2600 including a first contact layer 2601, a second contact layer 2602, and a first secondary layer 2603, and a second secondary layer 2604. The first contact layer 2601 and the second contact layer 2602 may vary as to thickness and the first secondary layer 2603 and the second secondary layer 2604 may also vary as to thickness. In various embodiments, the first secondary layer 2603 and the second secondary layer 2604 may be used separately or together to yield a desired thickness. Similarly, the first contact layer 2601 and the second contact layer 2602 may be used separately or together to yield a desired thickness, according to the needs of a particularly therapy.

### Methods of Use

In operation, a contact layer may be employed in treating a tissue site, for example, tissue having debris that may be desirably disrupted. For example, the tissue site may include biofilms, necrotic tissue, lacerated tissue, devitalized tissue, contaminated tissue, damaged tissue, infected tissue, exudate, highly viscous exudate, fibrinous slough, and/or other material that can generally be referred to as debris. Such debris may inhibit the efficacy of tissue treatment and slow the healing of the tissue site.

As an example, during treatment of a tissue site, a biofilm may develop on or in the tissue site. Biofilms may include a microbial infection that can cover a tissue site and impair healing of the tissue site. Biofilms can also lower the effectiveness of topical antibacterial treatments by preventing the topical treatments from reaching the tissue site. The presence of biofilms can increase healing times, reduce the efficacy and efficiency of various treatments, and increase the risk of a more serious infection. Additionally or alternatively, some tissue sites may not heal according to the normal medical protocol and may develop areas of necrotic tissue. Necrotic tissue may include dead tissue resulting from infection, toxins, or trauma that caused the tissue to die faster than the tissue can be removed by the normal body processes that regulate the removal of dead tissue. Sometimes, necrotic tissue may be in the form of slough, which may include a viscous liquid mass of tissue. Generally, slough is produced by bacterial and fungal infections that stimulate an inflammatory response in the tissue. Slough may be a creamy yellow color and may also be referred to as pus. Necrotic tissue may also include eschar. Eschar may be a portion of necrotic tissue that has become dehydrated and hardened. Eschar may be the result of a burn injury, gangrene, ulcers, fungal infections, spider bites, or anthrax. Conventionally, eschar may be generally difficult to move without the use of surgical cutting instruments.

In various embodiments, the debris may cover all or a portion of the tissue site. If the debris is at or in in the tissue site, the tissue site may be treated with various processes to disrupt the debris. Examples of disruption can include softening of the debris, separation of the debris from desired tissue, such as the subcutaneous tissue, preparation of the debris for removal from the tissue site, and removal of the debris from the tissue site.

In some embodiments, the diameter of the orifices may be selected to permit flow of debris through the orifices and associated apertures. For example, in some embodiments the diameter of the orifices may be selected based on the size of the debris to be lifted from the tissue site. Generally, larger orifices may allow larger debris to pass through the contact layer and smaller orifices may allow smaller debris to pass through the contact layer while blocking debris larger than the orifices. In some embodiments, successive applications of a contact layer can progressively smaller diameters of the orifices. Sequentially decreasing diameters of the orifices may also aid in fine-tuning a level of tissue disruption to the debris during the treatment of the tissue site. The diameter of the orifices can also influence fluid movement in the contact layer.

The contact layer may be prepared for use by selecting a desired orifice size from either the apertures of the first diameter range or the second diameter range and configuring the contact layer such that orifices of the desired orifice size may be contacted with the tissue site. In some embodiments, such as the in the embodiments of Figures 2, 3, 4, and 5, configuring the contact layer may include orienting the contact layer such that the orifices of a desired size may be disposed adjacent to the tissue site. For example, a user may orient the contact layer such that the orifices having a diameter in a first range are in contact with the tissue or such that the orifices having a diameter in a second range are in contact with the tissue site.

Additionally or alternatively, in some embodiments such as the embodiment of Figures 6 and 7, configuring the contact layer may include determining whether to remove the removable portions of the contact or to leave the removable portions fitted within the contact layer. For example, if the user desires to use the orifices having a diameter in the first range, the user may leave the removable portions within the contact layer or, if the user desires to use the orifices having a diameter in the second range, the user may remove the removable portions from the contact layer.

Additionally or alternatively, in some embodiments such as the embodiment of Figure 8, configuring the contact layer may include placing the contact layer in a conformation in which the orifices of a desired size may be placed in contact with the tissue site. With the contact layer configured for placement, the contact layer may be placed within, over, on, or otherwise proximate to a tissue site.

Additionally or alternatively, in some embodiments configuring the contact layer may include separating a portion of contact layer, such as along a perforation, to form a desired size or shape. In some embodiments, a portion of a first contact layer may be placed over a first portion of a tissue site and a portion of a second contact layer may be placed over a second portion of the tissue site. For example, utilizing portions from difference contact layers to cover different portions of a tissue site may be effective to disrupt debris at a first portion of the tissue site while the second portion of the tissue site remains relatively undisrupted.

In some embodiments, a cover may be placed over the contact layer and sealed to an attachment surface near the tissue site. For example, the cover may be sealed to undamaged epidermis peripheral to a tissue site. Thus, the dressing can provide a sealed therapeutic environment proximate to a tissue site, substantially isolated from the external environment, and the negative-pressure source can reduce the pressure in the sealed therapeutic environment. Negative pressure applied across the tissue site through the contact layer in the sealed therapeutic environment can induce macrostrain and microstrain in the tissue site, as well as remove exudates and other fluids from the tissue site, which can be collected in container.

In some embodiments, such as where the contact layer is employed to disrupt debris at a tissue site, the application of negative pressure to the tissue site via the contact layer can generate concentrated stresses in the debris adjacent and/or proximate to the apertures in the contact layer. The concentrated stresses can cause macro-deformations of the debris and the subcutaneous tissue, for example, which may draw portions of the debris and the subcutaneous tissue into the apertures. Similarly, the apertures of the contact layer may create macro-pressure points in portions of the debris and the subcutaneous tissue that are in contact with a tissue-facing surface of the contact layer, causing tissue puckering and nodules to be formed in the debris and the subcutaneous tissue. In some embodiments, formation of the nodules may lift debris and particulates off of the surrounding tissue, for example, operating in a piston-like manner to move debris toward and into the contact layer.

### Advantages

In various embodiments, a therapy system or components thereof, such as the contact layer, may be advantageously employed in the provision of therapy, such as negative pressure therapy, to a patient. For example, a contact layer configurable such that at least a portion of the apertures include a first plurality of orifices having a diameter in a first diameter range and such that at least a portion of the apertures include a second plurality of orifices having a diameter in a second diameter range may allow a user to choose an aperture size effective for obtaining desired results, such as the disruption of debris at the tissue site, in the context of the therapy. Thus, a single contact layer may be employed more effectively and efficiently across a wider variety of therapies.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the container 112 may be eliminated or separated from other components for manufacture or sale. In other example configurations, the controller 110 may also be manufactured, configured, assembled, or sold independently of other components.

## Claims

1. An apparatus (100) for treating a tissue site, the apparatus comprising:
a contact layer (108, 200, 300, 400, 500) formed from a compressible material, the contact layer (108, 200, 300, 400, 500) comprising a first surface (220, 320, 420, 520), a second surface (225, 325, 425, 525), and a plurality of apertures (205, 305, 405) extending at least partially through the contact layer (108, 200, 300, 400, 500),
at least a portion of the apertures (205, 305, 405) including a first plurality of orifices (210, 310, 410, 510) in the first surface (220, 320, 420, 520), each of the first plurality of orifices (210, 310, 410, 510) having a diameter in a first diameter range, wherein the first diameter range is from about 2 mm to about 6 mm,
at least a portion of the apertures (205, 305, 405) including a second plurality of orifices (215, 315, 415, 515) in the second surface (225, 325, 425, 515), each of the second plurality of orifices (215, 315, 415, 515) having a diameter in a second diameter range, wherein the second diameter range is from about 8 mm to about 15 mm;
a cover (106) configured to form a sealed space including the contact layer (108, 200, 300, 400, 500) and the tissue site, and
a reduced-pressure source (104) adapted to apply a reduced pressure to the sealed space,
wherein, in use, either the first surface (220, 320, 420, 520) or the second surface (225, 325, 425, 525) of the contact layer (108, 200, 300, 400, 500) contacts the tissue site.

2. The apparatus of claim 1, wherein the first plurality of orifices (210, 310, 410, 510) are grouped together and the second plurality of orifices (215, 315, 415, 515) are grouped together.

3. The apparatus of claim 1, wherein the first surface (220, 320, 420, 520) and the second surface (225, 325, 425, 526) are substantially planar.

4. The apparatus of claim 1, wherein each of the plurality of apertures (205, 305, 405) includes an orifice in the first diameter range and an orifice in the second diameter range.

5. The apparatus of claim 4, wherein the plurality of apertures (205, 305, 405) are conical.

6. The apparatus of claim 1, wherein the contact layer (108, 400, 500) comprises a first sublayer (401, 501) and a second sublayer (402, 502) joined to the first sublayer, the first sublayer and the second sublayer formed from the compressible material.

7. The apparatus of claim 1, wherein the compressible material comprises a thermoplastic elastomer.

8. The apparatus of claim 1, wherein the compressible material comprises a thermoplastic polyurethane.

9. The apparatus of claim 1, wherein the contact layer (108, 400, 500) is separable along a separation-line.

10. The apparatus of claim 9, wherein the separation-line includes perforations.

11. The apparatus of claim 9, wherein the separation-line is in the shape of a line, a circle, an ellipse, an oval, or combinations thereof.

## Patentansprüche

1. Eine Vorrichtung (100) zum Behandeln einer Gewebestelle, die Vorrichtung aufweisend:
eine Kontaktschicht (108, 200, 300, 400, 500), die aus einem komprimierbaren Material ausgebildet ist, die Kontaktschicht (108, 200, 300, 400, 500) aufweisend eine erste Oberfläche (220, 320, 420, 520), eine zweite Oberfläche (225, 325, 425, 525) und eine Mehrzahl von Öffnungen (205, 305, 405), die sich mindestens teilweise durch die Kontaktschicht (108, 200, 300, 400, 500) erstrecken,
wobei mindestens ein Teil der Öffnungen (205, 305, 405) eine erste Mehrzahl von Mündungen (210, 310, 410, 510) in der ersten Oberfläche (220, 320, 420, 520) einschließt, wobei jede der ersten Mehrzahl von Mündungen (210, 310, 410, 510) einen Durchmesser in einem ersten Durchmesserbereich umfasst, wobei der erste Durchmesserbereich von etwa 2 mm bis etwa 6 mm reicht,
mindestens ein Teil der Öffnungen (205, 305, 405) eine zweite Mehrzahl von Mündungen (215, 315, 415, 515) in der zweiten Oberfläche (225, 325, 425, 515) einschließt, wobei jede der zweiten Mehrzahl von Mündungen (215, 315, 415, 515) einen Durchmesser in einem zweiten Durchmesserbereich umfasst, wobei der zweite Durchmesserbereich von etwa 8 mm bis etwa 15 mm reicht;
eine Abdeckung (106), die konfiguriert ist, um einen abgedichteten Raum auszubilden, der die Kontaktschicht (108, 200, 300, 400, 500) und die Gewebestelle einschließt, und
eine Unterdruckquelle (104), die angepasst ist, um einen Unterdruck an den abgedichteten Raum bereitzustellen,
wobei in Verwendung entweder die erste Oberfläche (220, 320, 420, 520) oder die zweite Oberfläche (225, 325, 425, 525) der Kontaktschicht (108, 200, 300, 400, 500) die Gewebestelle kontaktiert.

2. Die Vorrichtung nach Anspruch 1, wobei die erste Mehrzahl von Mündungen (210, 310, 410, 510) zusammen gruppiert sind und die zweite Mehrzahl von Mündungen (215, 315, 415, 515) zusammen gruppiert sind.

3. Die Vorrichtung nach Anspruch 1, wobei die erste Oberfläche (220, 320, 420, 520) und die zweite Oberfläche (225, 325, 425, 526) im Wesentlichen eben sind.

4. Die Vorrichtung nach Anspruch 1, wobei jede der Mehrzahl von Öffnungen (205, 305, 405) eine Mündung in dem ersten Durchmesserbereich und eine Mündung in dem zweiten Durchmesserbereich einschließt.

5. Die Vorrichtung nach Anspruch 4, wobei die Mehrzahl der Öffnungen (205, 305, 405) konisch sind.

6. Die Vorrichtung nach Anspruch 1, wobei die Kontaktschicht (108, 400, 500) eine erste Unterschicht (401, 501) und eine mit der ersten Unterschicht verbundene zweite Unterschicht (402, 502) aufweist, wobei die erste Unterschicht und die zweite Unterschicht aus dem komprimierbaren Material ausgebildet sind.

7. Die Vorrichtung nach Anspruch 1, wobei das komprimierbare Material ein thermoplastisches Elastomer aufweist.

8. Die Vorrichtung nach Anspruch 1, wobei das komprimierbare Material ein thermoplastisches Polyurethan aufweist.

9. Die Vorrichtung nach Anspruch 1, wobei die Kontaktschicht (108, 400, 500) entlang einer Trennlinie trennbar ist.

10. Die Vorrichtung nach Anspruch 9, wobei die Trennlinie Perforationen einschließt.

11. Die Vorrichtung nach Anspruch 9, wobei die Trennlinie in der Form einer Linie, eines Kreises, einer Ellipse, eines Ovals oder Kombinationen davon vorliegt.

## Revendications

1. Appareil (100) permettant de traiter un site tissulaire, l'appareil comprenant :
une couche de contact (108, 200, 300, 400, 500) formée à partir d'un matériau compressible, la couche de contact (108, 200, 300, 400, 500) comprenant une première surface (220, 320, 420, 520), une seconde surface (225, 325, 425, 525) et une pluralité d'ouvertures (205, 305, 405) s'étendant au moins partiellement à travers la couche de contact (108, 200, 300, 400, 500),
au moins une partie des ouvertures (205, 305, 405) comportant une première pluralité d'orifices (210, 310, 410, 510) dans la première surface (220, 320, 420, 520), chacun parmi la première pluralité d'orifices (210, 310, 410, 510) ayant un diamètre dans une première plage de diamètre, dans lequel la première plage de diamètre va d'environ 2 mm à environ 6 mm,
au moins une partie des ouvertures (205, 305, 405) comportant une seconde pluralité d'orifices (215, 315, 415, 515) dans la seconde surface (225, 325, 425, 515), chacun parmi la seconde pluralité d'orifices (215, 315, 415, 515) ayant un diamètre dans une seconde plage de diamètre, dans lequel la seconde plage de diamètre va d'environ 8 mm à environ 15 mm ;
une couverture (106) conçue pour former un espace étanche comportant la couche de contact (108, 200, 300, 400, 500) et le site tissulaire, et
une source de pression réduite (104) adaptée pour appliquer une pression réduite à l'espace étanche,
dans lequel, en cours d'utilisation, soit la première surface (220, 320, 420, 520) soit la seconde surface (225, 325, 425, 525) de la couche de contact (108, 200, 300, 400, 500) vient en contact avec le site tissulaire.

2. Appareil selon la revendication 1, dans lequel la première pluralité d'orifices (210, 310, 410, 510) sont groupés ensemble et la seconde pluralité d'orifices (215, 315, 415, 515) sont groupés ensemble.

3. Appareil selon la revendication 1, dans lequel la première surface (220, 320, 420, 520) et la seconde surface (225, 325, 425, 526) sont sensiblement planes.

4. Appareil selon la revendication 1, dans lequel chacune parmi la pluralité d'ouvertures (205, 305, 405) comporte un orifice dans la première plage de diamètre et un orifice dans la seconde plage de diamètre.

5. Appareil selon la revendication 4, dans lequel la pluralité d'ouvertures (205, 305, 405) sont coniques.

6. Appareil selon la revendication 1, dans lequel la couche de contact (108, 400, 500) comprend une première sous-couche (401, 501) et une seconde sous-couche (402, 502) jointe à la première sous-couche, la première sous-couche et la seconde sous-couche étant formées à partir du matériau compressible.

7. Appareil selon la revendication 1, dans lequel le matériau compressible comprend un élastomère thermoplastique.

8. Appareil selon la revendication 1, dans lequel le matériau compressible comprend un polyuréthane thermoplastique.

9. Appareil selon la revendication 1, dans lequel la couche de contact (108, 400, 500) est séparable le long d'une ligne de séparation.

10. Appareil selon la revendication 9, dans lequel la ligne de séparation comporte des perforations.

11. Appareil selon la revendication 9, dans lequel la ligne de séparation est sous la forme d'une ligne, d'un cercle, d'une ellipse, d'un ovale, ou de combinaisons de ceux-ci.
